# FASCICULE DE BREVET EUROPEEN

(11) **EP 0 667 103 B1**
(45) Date de publication et mention de la délivrance du brevet: **08.09.1999**
(21) Numéro de dépôt: 95101581.7
(22) Date de dépôt: 07.02.1995
(51) Int. Cl.: A23C 11/10, A23L 1/20

(54) **Procédé d'extraction de matières solubles de fèves ou graines d'oléagineux**
Verfahren zur Extraktion löslichen Materials aus ölhaltigen Bohnen oder Samen
Process for extracting soluble substances from oleaginous seeds or beans

(30) Priorité: 15.02.1994 FR 9401840
(43) Date de publication de la demande: 16.08.1995
(73) Titulaire: De Stoutz, Jean-Christian, F-74500 Evian-Les-Bains (FR)
(72) Inventeur: De Stoutz, Jean-Christian, F-74500 Evian-Les-Bains (FR)
(74) Mandataire: Micheli & Cie

(56) Documents cités:
- FR-A- 2 366 798
- US-A- 3 288 614
- US-A- 4 369 198

## Description

La présente invention a pour objet un procédé d'extraction de matières solubles de fèves ou graines d'oléagineux et plus particulièrement un procédé de production de "lait" ou crèmes à base de telles graines oléagineuses.

Jusqu'ici le "lait" de soja est obtenu industriellement à partir de fèves de soja que l'on dépellicule au préalable puis qu'on trempe et fait macérer dans de l'eau additionnée notamment de bicarbonate de soude pendant dix à vingt-quatre heures au moins, suivant la saison et la température.

Par ces opérations, on réhydrate la graine de soja pour qu'elle puisse ensuite être écrasée, transformée en purée puis pressée et filtrée pour en extraire les matières solubles constituant du "lait" de soja. Ensuite, il est nécessaire de faire subir à cet extrait des traitements physiques et chimiques (notamment de solvant) pour éliminer les arômes indésirables, goût d'herbe, et les bactéries. En effet ces arômes indésirables et ces bactéries se sont développés dans la purée en raison du temps important de la macération des graines de soja. Il faut encore ensuite traiter à haute température le "lait" de soja obtenu pour être sûr de s'être débarrassé de tous les germes qui ont pu s'y développer.

Des procédés actuels pour l'obtention de lait ou de crèmes à partir de graines d'oléagineux pour les soins de la peau ou pour l'élaboration de produits cosmétiques sont similaires au procédé de fabrication du lait de soja décrit ci-dessus.

Ces techniques existantes nécessitent un équipement complexe, notamment pour la purification des produits obtenus, et l'intervention de main d'oeuvre qualifiée pour assurer la réalisation correcte de toutes ces opérations et finalement la qualité du produit obtenu.

La présente invention a notamment pour but la production de lait ou de crèmes à partir de graines oléagineuses.

La présente invention a pour objet un procédé d'extraction de matières solubles de fèves ou graines d'oléagineux, caractérisé par le fait que cette extraction a lieu en continu à partir de la fève ou graine brute, non trempée et non dépelliculée, qu'elle comporte un broyage de la graine brute sous pulvérisation d'un liquide suivi d'une filtration.

Le présent procédé de production de lait ou crème d'oléagineux est particulièrement intéressant car il permet d'utiliser la fève ou la graine brute, non trempée ou macérée et non dépelliculée ou non transformée en farine, c'est-à-dire telle qu'elle se présente après la récolte sans aucune opération de préparation.

De ce fait comme on le verra plus loin, il est possible de supprimer toute addition de produit chimique ou de solvant lors de la production du lait ou de la crème ce qui permet du même coup de supprimer toute opération antérieurement nécessaire au retrait de ces produits chimiques et à la purification du produit obtenu. De plus, comme on évite le trempage et la macération longue des graines, on évite toute prolifération de germes et de bactéries, de sorte que le lait obtenu peut être soumis à un traitement thermique à relativement basse température, suffisant pour en assurer la qualité et la conservation, mais beaucoup moins dénaturant qu'un traitement à haute température pour le produit fini.

Le présent procédé de production de lait ou de crème d'oléagineux comporte les opérations successives suivantes :
1. Les fèves ou graines d'oléagineux brutes de la récolte sont broyées finement en continu (particules inférieures à 1 millimètre) en présence d'une injection ou pulvérisation d'eau chaude. La température de l'eau pulvérisée est généralement comprise entre 70° et 100° C et le débit d'eau contrôlé est déterminé en fonction de la quantité de fèves broyées.
   Cette opération de broyage sous pulvérisation d'eau dure environ 1 à 3 secondes, de préférence 2 secondes.
2. Le broyat obtenu est immédiatement et continuellement filtré par centrifugation de manière à séparer les matières solides et liquides. Cette filtration est de préférence effectuée dans un filtre centrifuge conique. Pendant cette phase de filtration - centrifugation une couche de broyat se plaque contre la surface du filtre centrifuge conique, augmentant ainsi la finesse de filtration de ce filtre, et tandis que la partie liquide est récupérée par succion, la partie sèche ou pulpe est évacuée en continu par la force centrifuge. Une dépression est créée en fond de filtre par l'aspiration ou le pompage de l'eau qui a extrait les matières solubles, matières grasses, protéines, sels minéraux, vitamines, etc., et qui constitue le "lait" de soja ou d'autres graines d'oléagineux. Parallèlement à cette dépression en fond de filtre, se crée une surpression en haut du filtre provoquée par la remontée sous l'action de la force centrifuge de la pulpe qui est ensuite expulsée du filtre toujours par l'action de la force centrifuge.
   Cette aspiration au fond du filtre combinée à la surpression en haut du filtre provoque un phénomène de déaération automatique du filtrat.
   C'est au cours de cette phase du procédé de production qui dure environ une dizaine de secondes (de 8 à 20 secondes par exemple) que d'une part les matières utiles sont extraites des graines et, d'autre part, l'ensemble des matières non solubles tels que les résidus de pulpe, de fibres de cellulose et la pellicule externe des graines de même que de divers déchets tels que micro-cailloux, sables, etc., sont évacués.
   Ainsi l'ensemble de ces deux premières opérations, broyage-pulvérisation et filtration-centrifugation, ne dure que quelques secondes, de 8 à 20 généralement, mais de préférence entre 10 et 15 secondes.
   Grâce à cette très courte durée de contact entre la graine et l'eau (environ 12 secondes contre 10 à 24 heures de macération dans les procédés connus) on évite au produit, de se charger du goût désagréable de fève dû à l'action d'enzyme, notamment de la lipoxygénase, car pendant une aussi courte période cet enzyme n'a pas le temps d'agir. De même, grâce toujours à cette très courte durée de contact des graines avec l'eau qui est de préférence à 80°C, on obtient une réduction considérable de la charge bactérienne ainsi que de la quantité de facteurs anti-nutritionnels dans le cas de produits alimentaires (facteurs anti-trypsique).
3. Dans une troisième opération qui suit immédiatement les deux premières et qui s'effectue également en continu le filtrat ainsi obtenu, le lait est soumis à un traitement thermique à basse température pour sa pasteurisation et sa conservation.
   La basse température, 70° à 100°C (de préférence de 90° à 100°C) de ce traitement thermique, rendue possible par la faible charge bactérienne du filtrat, permet une réduction notable de la dénaturation des bonnes protéines, un meilleur respect de la teneur en vitamines et donc finalement l'obtention d'un "lait" de soja d'une plus grande valeur nutritionnelle que celui obtenu par les procédés habituels ou d'une façon général d'un produit par exemple un lait cosmétique de meilleure qualité que ceux obtenus antérieurement.
   Les diagrammes qui suivent sont le résultat de tests comparatifs entre du "lait" de soja produit selon le présent procédé sans pré-trempage (S.P.T.), un "lait" de soja préexistant et du lait de vache.

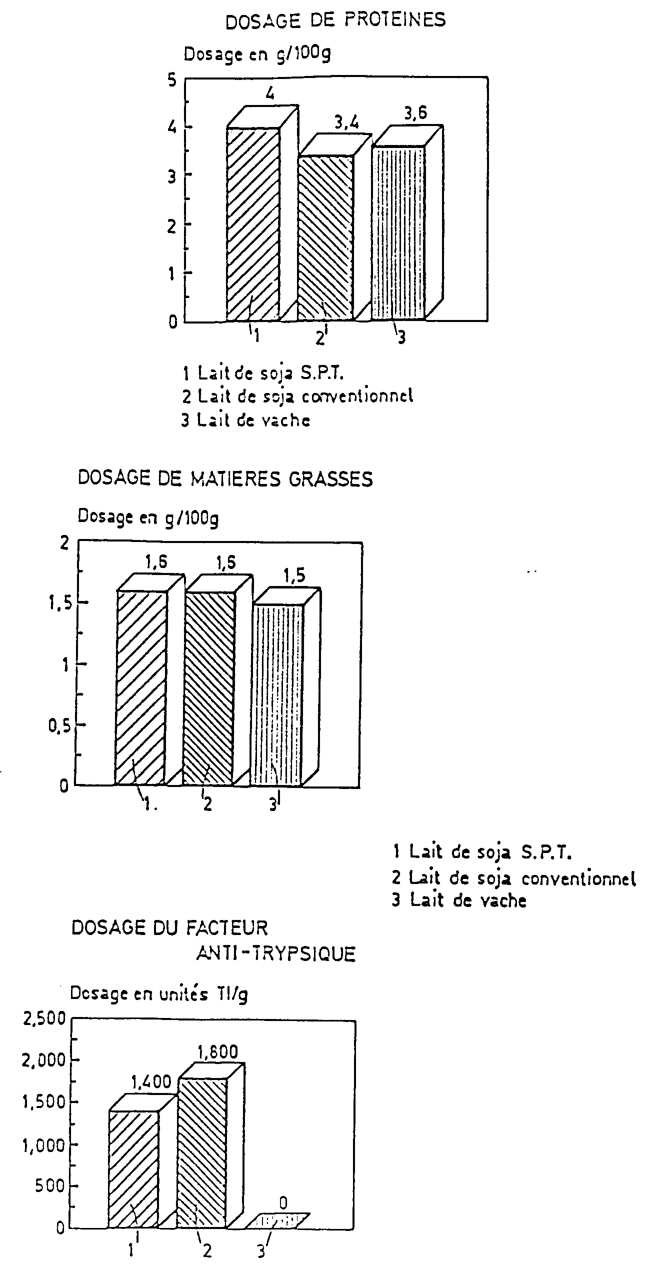

Ces diagrammes montrent à l'évidence que le "lait" de soja selon le présent procédé S.P.T. présente un meilleur dosage de protéines, un dosage de matières grasses légèrement supérieur au lait de vache et un dosage du facteur anti-trypsique nettement inférieur au "lait" de soja conventionnel.

En plus des avantages et des qualités supérieures du "lait" de soja ou du lait de graines oléagineuses obtenu par le présent procédé de production, ce procédé réduit les dépenses énergétiques du fait du traitement thermique à plus basse température et à l'absence de traitements chimiques.

Ce procédé est considérablement plus simple dans sa mise en oeuvre que les procédés existants car il n'y a plus de trempage et de macération, les graines sont utilisées brutes de la récolte, tous les traitements chimiques sont supprimés, et le processus est continu du début à la fin de la production.

On peut ainsi avec ce procédé de production utiliser de petites unités de production délocalisées dont le fonctionnement est assuré par un personnel non spécialisé. Ces petites unités de production nécessitent un investissement réduit et peuvent être implantées directement dans des zones ou villages touchés par la malnutrition dans des pays à plus faible technologie mais justement producteurs de soja.

Dans ce qui précède on a principalement parlé de la production de "lait" de soja mais il va de soi que ce procédé de production est utilisable pour toute extraction de matières solubles, de fèves ou graines d'oléagineux notamment destinée à la fabrication de produits de soins pour la peau ou de produits cosmétiques.

La principale originalité de ce procédé réside dans le fait que les fèves ou graines sont utilisées brutes, sans dépelliculage ni trempage et que la durée de contact entre la fève ou la graine, et notamment sa pellicule externe, est réduite, inférieure a 30 ou 45 secondes, et de préférence de l'ordre de 10 à 15 secondes.

Il est évident que pour la bonne conservation des produits obtenus, lait de soja ou laits et crèmes pour les soins de la peau, il peut être nécessaire de faire subir à ces produits un traitement thermique. Ce traitement thermique peut avantageusement être réalisé par effet Joule selon la technique décrite dans le brevet EP 0.476.311.

## Revendications

1. Procédé d'extraction de matières solubles de fèves ou graines d'oléagineux, caractérisé par le fait que cette extraction a lieu en continu à partir de la fève ou graine brute, non trempée et non dépelliculée, qu'elle comporte un broyage de la graine brute sous pulvérisation d'un liquide suivi d'une filtration.

2. Procédé selon la revendication 1, caractérisé par le fait que le broyage de la fève ou de la graine brute s'effectue sous pulvérisation d'un liquide chaud; que la filtration s'effectue par centrifugation et par le fait que la durée de contact entre la fève ou son broyat et le liquide est inférieure à 1 minute.

3. Procédé selon la revendication 2, caractérisé par le fait que les particules de fèves ou de graines broyées sont de l'ordre du millimètre et que le liquide pulvérisé pendant le broyage est de l'eau à une température comprise entre 70 et 100° C, de préférence de 90° à 100°C.

4. Procédé selon l'une des revendications 1 à 3, caractérisé par le fait que l'extraction des matières solubles est réalisée pendant la filtration, la partie liquide étant aspirée, et que simultanément les déchets sont expulsés du filtre par débourrage.

5. Procédé selon la revendication 4, caractérisé par le fait que l'aspiration du filtrat s'effectue en partie basse du filtre tandis que l'expulsion des déchets est effectuée par le haut du filtre, cette aspiration et cette expulsion créant dans le filtre un gradient de pression provoquant un dégazage.

6. Procédé selon l'une des revendications précédentes, caractérisé par le fait que la durée de contact du liquide avec la fève ou la graine est inférieure à 30 secondes, de préférence comprise entre 10 et 15 secondes.

7. Procédé selon l'une des revendications précédentes, caractérisé par le fait que les opérations de broyage-pulvérisation et de filtration-centrifugation sont suivies d'un traitement thermique à une température inférieure à 120° C.

8. Lait de graine oléagineuse obtenu par procédé selon l'une des revendications précédentes.

9. Lait selon la revendication 8 obtenu à partir de graine de soja.

10. Lait selon la revendication 8 pour les soins de la peau, ou lait cosmétique obtenu à partir de graines oléagineuses.

## Claims

1. A process for extracting soluble material from beans or seeds from oleaginous plants, characterized in that this extraction is carried out continuously from raw beans or seeds without soaking or dehulling, and includes a grinding of the raw seeds while spraying with a liquid, followed by a filtering.

2. A process according to claim 1, characterized in that the grinding of the beans or of the seeds is carried out while spraying with a hot liquid; in that the filtering is carried out by centrifugation and in that the contact time between the beans or the ground material and the liquid is lesser than 1 minute.

3. A process according to claim 2, characterized in that the particles of the ground beans or seeds have a size in the order of one millimeter and that the liquid sprayed during the grinding is water at a temperature comprised between 70 ° and 100 °C and, preferably, from 90 ° to 100 °C.

4. A process according to claims 1 to 3, characterized in that the extraction of the soluble materials is carried out during the filtering, the liquid part being sucked away and in that, simultaneously, the residues are forcibly expelled from the filter.

5. A process according to claim 4, characterized in that the suction of the filtrate is carried out from the lower part of the filter, whereas the expelling of the residues is carried out from the upper part of the filter, this suction and this expulsion creating in the filter a pressure gradient resulting in a degassing.

6. A process according to one of the preceding claims, characterized in that the contact time of the liquid with the beans or seeds is lesser than 30 seconds and is preferably comprised between 10 and 15 seconds.

7. A process according to one of the preceding claims, characterized in that the grinding-spraying and the filtering-centrifugating operations are followed by a thermal treatment at a temperature lesser than 120 °C.

8. Milk from oleaginous beans obtained by a process according to one of the preceding claims.

9. Milk according to claim 8, obtained from soy beans.

10. Milk according to claim 8 for skin care purposes or for making a cosmetic milk obtained from oleaginous beans.

## Patentansprüche

1. Verfahren zur Extraktion löslichen Materials aus ölhaltigen Bohnen oder Samen, dadurch gekennzeichnet, dass diese Extraktion kontinuierlich und mit den rohen, nicht eingeweichten und nicht geschälten Bohnen oder Samen erfolgt und dass sie ein Mahlen der rohen Samen unter Zerstäubung einer Flüssigkeit, gefolgt von einer Filtration umfasst.

2. Verfahren gemäss Anspruch 1, dadurch gekennzeichnet, dass das Mahlen der rohen Bohnen oder Samen unter Zerstäubung einer heissen Flüssigkeit erfolgt; dass die Filtration durch Zentrifugieren erfolgt; und dass die Berührungszeit zwischen den Bohnen oder gemahlenen Bohnen und der Flüssigkeit weniger als eine Minute beträgt.

3. Verfahren gemäss Anspruch 2, dadurch gekennzeichnet, dass die gemahlenen Bohnen- oder Samenteilchen etwa millimetergross sind und die während des Mahlens zerstäubte Flüssigkeit Wasser bei einer Temperatur zwischen 70 und 100 °C, bevorzugt zwischen 90 und 100 °C ist.

4. Verfahren gemäss einem der Ansprüche 1 bis 3, dadurch gekennzeichnet, dass die Extraktion löslichen Materials während der Filtration erfolgt, wobei der flüssige Anteil abgesaugt wird, während die Abfälle gleichzeitig durch Abstreifen vom Filter entfernt werden.

5. Verfahren gemäss Anspruch 4, dadurch gekennzeichnet, dass das Absaugen des Filtrats im unteren Abschnitt des Filters erfolgt, während die Entfernung der Abfälle vom oberen Teil des Filters her erfolgt, wobei dieses Absaugen und dieses Entfernen im Filter einen Druckgradienten erzeugen, der eine Entgasung hervorruft.

6. Verfahren gemäss einem der vorangehenden Ansprüche, dadurch gekennzeichnet, dass die Berührungszeit der Flüssigkeit mit den Bohnen und Samen weniger als 30 Sekunden beträgt und bevorzugt zwischen 10 und 15 Sekunden liegt.

7. Verfahren gemäss einem der vorangehenden Ansprüche, dadurch gekennzeichnet, dass auf die Arbeitsgänge von Mahlen und Zerstäuben sowie von Filtrieren und Zentrifugieren eine Wärmebehandlung bei einer Temperatur unterhalb von 120 °C folgt.

8. Durch ein Verfahren gemäss einem der vorangehenden Ansprüche gewonnene Ölsamenmilch.

9. Ausgehend von Sojabohnen gewonnene Milch gemäss Anspruch 8.

10. Milch gemäss Anspruch 8 zur Hautpflege oder ausgehend von Ölsamen gewonnene kosmetische Milch.
